# EUROPEAN PATENT APPLICATION

(11) **EP 4 458 980 A1**
(43) Date of publication of application: **06.11.2024**
(21) Application number: 22916109.6
(22) Date of filing: 27.12.2022
(51) Int. Cl.: C12P 21/00

(54) **METHOD FOR PRODUCING PROTEIN IN BACULOVIRUS EXPRESSION SYSTEM**

(30) Priority: 27.12.2021 JP 2021213132
(71) Applicant: Denka Company Limited, Tokyo 103-8338 (JP)
(72) Inventor: NARAHARA, Tomohiro, Gosen-shi, Niigata 959-1695 (JP); MITSUMATA, Ryotaro, Gosen-shi, Niigata 959-1695 (JP)
(74) Representative: Wächtershäuser & Hartz Patentanwaltspartnerschaft mbB
(86) International application number: PCT/JP2022/048243
(87) International publication number: WO 2023/127887

(57) **Abstract**

Provided is a method for producing a protein expressed with excellent efficiency in the baculovirus expression system. A method for producing a protein in a baculovirus expression system, comprising a step of adding a calcium salt to a culture medium after a recombinant baculovirus is inoculated into an insect cell.

## Description

### Technical Field

The present invention relates to a method for producing a protein in the baculovirus expression system.

### Background Art

The baculovirus expression system is a technology for expressing a protein of interest by infecting an insect cell with a baculovirus having a gene of the protein of interest integrated therein. The protein expressed in the insect cell undergoes post-translational modification, with the result that a protein having the same physiological activity as in a mammalian can be obtained. For this reason, the baculovirus expression system is superior to the protein expression system of prokaryotes such as *Escherichia coli.* Because of this, the baculovirus expression system has been widely used also as a technique for producing biologics such as a vaccine and a commercial-scale mass culture technique has been established. Furthermore, since humans are not infected with a baculovirus, the baculovirus is low in risk and easy to handle. This is also a reason why a baculovirus has been widely used.

Examples of the protein produced by the baculovirus expression system include a cervical cancer vaccine. Since it is difficult to artificially culture human papillomavirus, which is a causative virus for cervical cancer, it is impossible to proliferate the virus itself and eliminate infectivity to produce an inactivated vaccine thereof. However, it is known that when capsid protein L1 is expressed in the baculovirus expression system, it forms a virus-like particle (VLP). Since it lacks a viral genome, the VLP has no infectivity but the external structure thereof is highly analogous to that of a virus. Thus, VLP is useful as a vaccine antigen. Vaccine production using the baculovirus expression system has been applied to the production of other vaccines such as an influenza vaccine and a norovirus vaccine (under development).

To increase a protein yield in the baculovirus expression system, it is commonly known that infection is carried out with an enhanced multiplicity of infection (MOI) and at high titer. However, high-MOI infection requires a large amount of a recombinant baculovirus serving as a seed. Thus, it was necessary to previously prepare a large amount of recombinant baculovirus as a seed for producing a protein. That is, efficiently obtaining a protein with a low MOI infection is an object to be accomplished in the baculovirus expression system. For example, in the method of Patent Literature 1, a protein of interest is efficiently obtained with an MOI of less than 1. However, the scale of a culture vessel in this case is as large as 2 to 250 L and thus a proper culturing facility is required.

If a protein of interest remains within a host insect cell, the cell is sometimes disrupted to release the protein of interest into the culture supernatant. In the case, physical disruption by, e.g., a homogenizer, or a surfactant is used but there is a high possibility of denature not only the insect cell but also the high-order structure of the protein of interest expressed. As an improving means, Patent Literature 2 discloses omitting a cell-disruption step by extending a culture period until the number of dead cells increases and a protein of interest is released into the culture supernatant. However, the extension of the culture period extends a production period and, in addition, an adverse effect of, e.g., a protease derived from dead cells on a protein of interest is concerned.

### Citation List

### Patent Literatures

Patent Literature 1: JP-A-2003-533964
Patent Literature 2: JP-A-2015-15931

### Summary of Invention

### Technical Problem

The present invention relates to a method for producing a protein expressed with excellent efficiency in the baculovirus expression system.

### Solution to Problem

The inventors investigated culture conditions for insect cells to efficiently express a protein of interest in the baculovirus expression system. As a result, they found that the expression level of the protein of interest increases by adding a calcium salt to a culture medium after inoculation of a recombinant baculovirus.

That is, the present invention relates to the following 1) to 4).
1) A method for producing a protein in a baculovirus expression system, comprising a step of adding a calcium salt to a culture medium after a recombinant baculovirus is inoculated into an insect cell.
2) The method according to 1), wherein the calcium salt is added so as to increase a concentration of calcium in a culture supernatant by from 25 to 250 µM as a final concentration.
3) The method according to 1) or 2), wherein the calcium salt is added between 12 and 36 hours after the inoculation of the baculovirus.
4) The method according to any one of 1) to 3), wherein the calcium salt is calcium chloride.

### Advantageous Effects of Invention

According to the present invention, it is possible to efficiently produce a protein of interest by the baculovirus expression system. That is, a protein of interest can be produced without unnecessarily increasing a multiplicity of infection (MOI) and without extending the culture period.

### Brief Description of Drawings

Figure 1: Viable insect-cell rate when calcium chloride is added.
Figure 2: Evaluation of production yield of a protein of interest when calcium chloride is added.
Figure 3: Evaluation of production yield of a protein of interest depending on calcium-chloride concentration.

### Description of Embodiments

Now, preferred embodiments of the present invention will be described in detail. However, the present invention is by no means restricted to the following embodiments.

The method for producing a protein of the present invention is a method for producing a protein in a baculovirus expression system, comprising a step of adding a calcium salt to a culture medium after a recombinant baculovirus is inoculated into an insect cell.

In the present invention, the "baculovirus expression system" refers to an expression system, in which an inset cell is infected with a recombinant baculovirus containing the gene for a protein that is of interest (referred to also as a "protein of interest") and in which the protein of interest is expressed.

The "recombinant baculovirus" is a baculovirus virus (baculovirus vector) prepared by integrating the gene of a protein of interest downstream of a promoter (polyhedrin promoter) enabling expression of the gene in cultured cells of the insect.

Here, the type of baculovirus is not particularly limited as long as it is a virus capable of transmitting to a lepidopteran insect or a cultured cell of the insect but preferably a nucleopolyhedrovirus (NPV) or a modified virus thereof. Examples of such a virus include BmNPV, HycuNPV, AnpeNPV and AcNPV.

The recombinant baculovirus can be prepared by a method known in the technical field. Examples of such a method include a method using a transfer vector which can insert a desired gene into baculovirus DNA by homologous recombination within a lepidopteran insect. In this method, cultured cells of a lepidopteran insect are cotransfected with the transfer vector having the gene of a protein of interest integrated therein and linearized baculovirus DNA with, e.g., a restriction enzyme, and infected cells are screened to obtain a recombinant baculovirus.

Other than this, there is a method for preparing baculovirus DNA having a desired gene inserted therein (recombinant bacmid DNA) by homologous recombination in a bacterium. In this method, bacteria previously containing a baculovirus gene are transformed with a transfer vector having the gene of a protein of interest integrated therein to obtain recombinant DNA, and then, cultured cells of the lepidopteran insect are transfected with the recombinant DNA, whereby a recombinant baculovirus can be obtained. Here, examples of the bacterium to be used herein include, *E. coli* strain and descendent stains therefrom.

The transfer vector to be used in such a case is not particularly limited as long as it is a vector DNA which has a promoter enabling gene expression in a lepidopteran insect or a cultured cell of the insect and to which a desired gene can be inserted downstream of the promoter. Such a transfer vector itself is commonly known in the technical field. Examples of the vector include pM02, pM23, pCPM, pYNG, pBM030, pBM050, pVL1392, pPSC8 and pFastBac. Here, the promoter may be appropriately selected from promoters commonly known in the technical field. Examples of the promoter include a polyhedrin promoter, a p10 promoter and a silkworm actin promoter.

A method using the bacmid is commercially available in a kit form. Examples of the kit known include Bac-to-Bac system (Thermo Fisher Scientific), BaculoGold system (BD Biosciences), SuperBAC system (SHEATECH), BacPAC system (Clontech), flashBAC system (Oxford Expression), BacMagic system (Merck), BestBac system (Expression Systems) and BacuVance system (GenScript).

Here, examples of the commercially available baculovirus vector known in the technical field include ProFold vector (AB Vector), ProEasy vector (AB Vector), FoldHelper vector (AB Vector), pVL vector (AB Vector), pAc vector (AB Vector), pAB vector (AB Vector), pIEx vector (Merck), pBAC vector (Merck) and pTriEx vector (Merck) .

In the present invention, the gene of a protein of interest to be integrated into a recombinant baculovirus is not particularly limited. The protein of interest may be a monomer or a protein forming a complex such as VLP. Examples of the protein of interest include a functional protein to be used as biopharmaceuticals such as vaccines and antibodies, clinical test reagents, foods and cosmetics.

In the present invention, the "insect" refers to a lepidopteran insect suitable for expression of a recombinant protein. Examples of the insect include silkworm (*Bombyx mori*), *Spilosoma imparilis, Antheraea pernyi, Spodoptera frugiperda* and *Trichoplusiani.*

The lepidopteran insect cell to be cultured is not particularly limited as long as it is a cell line established from the lepidopteran insect. Examples of the insect cell include silkworm cells (e.g., BmN cell, BmN4 cell and BoMo cell), *Antheraea pernyi* cells (Anpe cells), *Spodoptera frugiperda* cells (e.g., Sf9 cell and Sf21 cell), *Spilosoma imparilis* cells (SpIm cells) and *Trichoplusiani* cells (e.g., Tn-5 cell, HIGH FIVE cell and MG1 cell). Further, modified insect cell strains obtained by modifying these cells are included.

Inoculation (infection) of a recombinant baculovirus into an insect cell is carried out after preliminarily proliferating the insect cell.

The culture may be either adherent culture or suspension culture, preferably suspension culture.

Examples of the culture medium to be used include mediums commonly used, such as TNM-FH medium (manufactured by Pharmingen), Express Five SFM (Thermo Fisher Scientific), Sf-900 II SFM (Thermo Fisher Scientific), Sf-900 III (Thermo Fisher Scientific), ExpiSf CD (Thermo Fisher Scientific), IS Sf Insect medium (FUJIFILM Irvine Scientific), 4Cell Insect CD medium (SARTRIUS), Insect-XPRESS medium (LONZA), ESF 921 medium (Expression Systems) and ESF SF medium (Expression Systems). As a case requires, an antibiotic substance such as penicillin, streptomycin or gentamicin may be added to the culture medium.

The culture temperature is preferably from 25 to 30°C, more preferably from 26 to 28°C, further preferably 27°C.

The cell concentration is preferably from 1 × 10⁵ to 1 × 10⁷ cells/mL, more preferably from 2 × 10⁵ to 5 × 10⁶ cells/mL, further preferably from 5 × 10⁵ to 2 × 10⁶ cells/mL.

Infection of the cultured cells with a recombinant baculovirus is carried out by inoculation through the addition of a solution containing the recombinant baculovirus to a culture medium when the insect-cell concentration reaches a value from 1 × 10⁵ to 1 × 10⁷ cells/mL.

Here, the viral inoculum dose in terms of MOI is, for example, from 0.01 to 20 and preferably from 0.1 to 1.

In the present invention, a calcium salt is added to the culture medium in the step of infecting cultured cells with a recombinant baculovirus, whereby the concentration of a calcium ion in the culture supernatant is increased.

Here, the "calcium salt" refers to a salt containing a calcium ion (Ca²⁺) as a constituent ion. Examples of the salt include calcium chloride (CaCl₂), calcium fluoride (CaF₂), calcium bromide (CaBr₂), calcium iodide

(CaI₂), calcium oxide (CaO), calcium hydride (CaH₂), calcium hydroxide (Ca(OH)₂), calcium carbide (CaC₂), calcium phosphide (Ca₃P₂), calcium carbonate (CaCO₃), calcium hydrogen carbonate (Ca(HCO₃)₂), calcium nitrate (Ca(NO₃)₂), calcium nitrite (Ca(NO₂)₂), calcium sulfate (CaSO₄), calcium sulfite (CaSOs), calcium hydrogen sulfate (Ca(HSO₄)₂), thiocalcium sulfate (CaS₂O₃), calcium phosphate (Ca(PO₄)₂), calcium hypochlorite (Ca(ClO)₂), calcium chlorate (Ca(ClO₃)₂), calcium perchlorate (Ca(ClO₄)₂), calcium bromate (Ca(BrO₃)₂), calcium iodate (Ca(IO₃)₂), calcium arsenate (Ca(AsO₄)₂), calcium metasilicate (CaSiO₃), calcium permanganate (Ca(MnO₄)₂), calcium chromate (CaCrO₄), calcium dichromate (CaCr₂O₇), calcium tetraborate (CaB₄O₇), calcium cyanide (Ca(CN)₂), calcium carbonate magnesium (CaMg(CO₃)₂), calcium phosphate hydroxide (Ca₅(PO₄)₃(OH)), calcium disodium ethylenediaminetetraacetate (CaNa₂C₁₀H₁₂N₂O₈), calcium acetate (Ca(CH₃COO)₂), calcium citrate (Ca₃(C₆H₅O₇)₂) , calcium ascorbate (CaC₁₂H₁₄O₁₂), calcium gluconate (CaC₁₂H₂₂O₁₄), saccharin calcium (CaC₁₄H₈N₂O₆S₂), carboxymethylcellulose calcium (Ca.xC₂H₄O₃. x: unspecified), calcium glutamate (CaC₁₀H₁₆N₂O₈), calcium stearate (CaC₃₆H₇₀O₄) and calcium stearoyl lactate (CaC₄₂H₇₈O₈). Here, the state of the calcium salt to be added is not restricted.

The timing of adding a calcium salt is not particularly limited. For example, a calcium salt may be added between 0 and 48 hours after the vial inoculation, preferably between 12 and 36 hours after the vial inoculation.

The calcium salt is added so as to increase a concentration of calcium (concentration of calcium ion) in the culture supernatant by a range of 1 µM or more, preferably 10 µM or more, and more preferably 25 µM or more, and 1 000 µM or less, preferably 500 µM or less, and more preferably 250 µM or less, or by a range of from 1 to 1 000 µM, preferably from 10 to 500 µM, and more preferably from 25 to 250 µM, as a final concentration. When the calcium salt is added in an amount within the above range, the expression of a protein can be improved without decreasing a viable cell rate.

A protein of interest is produced in a cell by culturing the cell for from 1 to 7 days, preferably from 2 to 4 days, more preferably 4 days after infection (inoculation with a virus). Here, during the culture, a protease inhibitor and/or a nuclease can be appropriately added.

The protein of interest thus produced can be isolated and purified by a method usually used for the isolation and purification of proteins. For example, in a case where the protein of interest is expressed within a cell and dissolved, cells are centrifugally collected after the completion of culture, suspended in an aqueous buffer solution, and disrupted by, e.g., a sonicator, French press, a Manton Gaulin homogenizer or Dinomill, to obtain a cell-free extract. The cell-free extract is centrifuged to obtain the supernatant. A purified product can be obtained from the supernatant using a usual method for isolation and purification of a protein, namel, a solvent extraction method, a salting-out method, a desalination method, a precipitation method with an organic solvent, an anion exchange chromatography method, a cation exchange chromatography method, a hydrophobic chromatography method, an ultrafiltration method, a gel filtration method, an affinity chromatography method, a chromate-focusing method, electrophoresis and immunoprecipitation, alone or in combination.

Further, the protein of interest is expressed and forms an insoluble material in a cell, cells are collected, disrupted and centrifuged in the same manner as above. The insoluble material of the protein of interest was recovered as a precipitation fraction. The collected insoluble material of a protein of interest can be solubilized with a protein denaturant. After the operation, a purified product of the protein of interest can be obtained by the same isolation and purification method as above.

If the protein of interest is released to the outside of the cells, the cells are removed after the completion of culture by, e.g., centrifugation and filtration, to obtain the culture supernatant containing the protein of interest. After the operation, a purified product of the protein of interest can be obtained by the same isolation and purification method as above.

### Examples

Now, the present invention will be more specifically described with reference to Examples, however, the present invention is by no means restricted thereto. Reference Example 1: Evaluation of toxicity of calcium chloride to insect cells

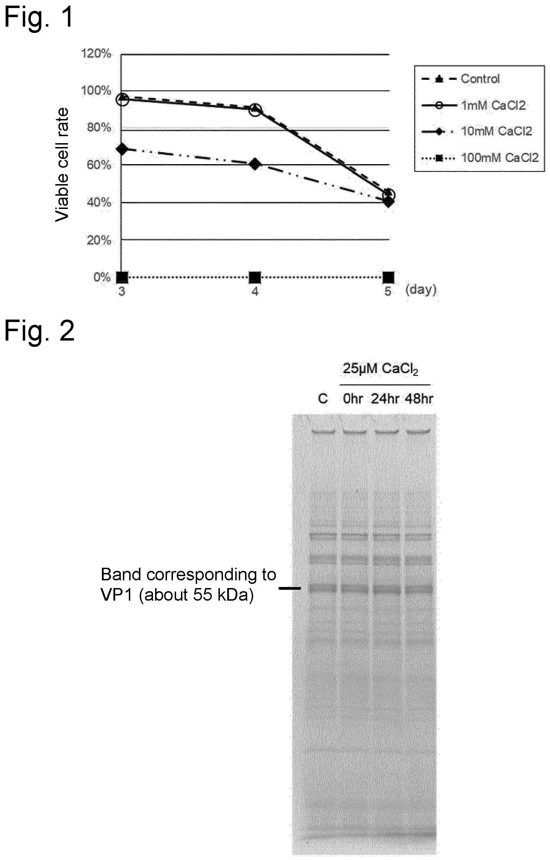

High Five cells (Thermo Fisher Scientific, Express Five^{™} SFM) were seeded at a seeding concentration of 1 × 10⁶ cells/mL so as to satisfy a culture scale of 30 mL. Twenty-four hours after the initiation of culture, calcium chloride was added so as to increase a final concentration by 1, 10 and 100 mM. Here, a calcium chloride-free group was used as a control. From day 3 to 5, the cell culture solution was sampled and stained with a 0.2% trypan blue solution. The numbers of viable cells and dead cells were counted by a hemocytometer. From the total cell count and the viable-cell count, the rate of viable cells was calculated. The results are shown in Figure 1.

As shown in Figure 1, it was confirmed that the viable cell rate was the same as in the control at 1 mM from day 3 to 5, and the viable cell rate was low at 10 and 100 mM on day 3. Thus, as long as the concentration of calcium chloride is 1 mM or lower, calcium chloride is not cytotoxic, and it is considered that the protein expression is not impaired.

### Example 1: Evaluation of production yield of a protein of interest when calcium chloride is added

High Five cells (Thermo Fisher Scientific, Express Five^{™} SFM) were seeded at a seeding concentration of 1 × 10⁶ cells/mL so as to satisfy a culture scale of 30 mL. The culture solution was infected with a recombinant baculovirus prepared by integrating a gene sequence of a VP1 protein of norovirus at an MOI = 0.1. Calcium chloride was added at the time points of 0, 24 and 48 hours after infection so as to increase a final concentration by 25 µM, and then, cell culture was continued. Note that, cell culture was carried out in a calcium chloride-free condition and used as a negative control. On Day 3 after the viral infection, Benzonase (Merck) was added so as to obtain a final concentration of 50 U/mL and 30 mL of protease inhibitor tablets (Thermo Fisher Scientific) was added, and then, cell culture was continued. On Day 4 after the viral infection, a cell culture solution was collected and centrifuged at 1 000 × g for 15 minutes, and then, the supernatant was collected. The supernatant collected was centrifuged at 11 000 × g for 60 minutes and the supernatant was collected. The supernatant and a sample buffer (8% SDS, 40% glycerol/250 mM Tris-HCl Buffer pH6.8) were mixed in the same amounts, and further, 1/10 volume of 1 mg/mL BPB was mixed. The resultant solution was reacted at 95°C for 5 minutes and used as an SDS-PAGE sample.

The SDS-PAGE was carried out by an electrophoresis device (ATTO CORPORATION) having 12.5% polyacrylamide gel (ATTO CORPORATION) set therein. The device was filled with a running buffer (0.1% SDS, 192 mM glycine/25 mM Tris Buffer pH8.3), and SDS-PAGE samples were electrophoresed. After the completion of electrophoresis, the gel was stained with Bio-Safe Coomassie Stain (Bio-Rad) for one hour, decolored by ultra-pure water, and subjected to densitometric analysis by an image analyzer (Bio-Rad).

Before the densitometric analysis, a calibration curve was prepared using VP1 having a preliminarily controlled concentration as a standard. The amounts of bands corresponding to VP1 of individual samples were calculated relative to the condition in the absence of calcium chloride. From the analysis results (Figure 2 and Table 1), when calcium chloride is added 24 hours after the initiation of culture, the yield of a protein of interest was improved by about 10%. Since the doubling time of insect cells is about 24 hours, it is considered that the insect cells are provably once divided after viral infection. In other words, when calcium chloride is added between 12 and 36 hours after viral infection during which most of the cells undergo a first cell division, it was considered that the yield of a protein of interest can be improved most efficiently.

**[Table 1]**

| Specimen | Negative control | 0 hr | 24 hr | 48 hr |
|---|---|---|---|---|
| VP1 band intensity (relative value) | 100% | 102% | 111% | 102% |

Example 2 Evaluation of production yield of a protein of interest depending on calcium-chloride concentration

A High Five cell culture solution in which cells were seeded at a seeding concentration of 1 × 10⁶ cells/mL so as to satisfy a culture scale of 30 mL, was infected with a recombinant baculovirus prepared by integrating a gene sequence of a norovirus VP1 protein, at MOI = 0.5. On Day 1 after the initiation of culture, calcium chloride was added so as to increase a final concentration by 2.5, 25, 250, 500 and 1 000 µM, and then, the cell culture was continued. On day 3, Benzonase (Merck) was added so as to be a final concentration of 50 U/mL and 30 mL of protease inhibitor tablets (Thermo Fisher Scientific) was added, and then, the cell culture was continued. On day 4, the culture supernatant was collected in the same manner as in Example 2 and subjected to Western Blotting.

For Western Blotting, after electrophoresis was carried out in the same manner as in Example 1, a transfer reaction was carried out from the gel to PVDF membrane (Merck) by a semi-dry transfer device (ATTO CORPORATION). After the transfer reaction, the PVDF membrane was soaked in a 10% skim milk-containing TBS and incubated at room temperature for two hours. After the PVDF membrane was washed three times with an appropriate amount of TBS and soaked in an anti-norovirus VP1 protein antibody (manufactured by the inventors) diluted with a 1% skim milk-containing TBST and incubated at 4°C for from 18 to 20 hours. The PVDF membrane was washed five times with an appropriate amount of TBST, soaked in a HRP-labeled anti-mouse antibody (Jackson Immuno Research Laboratories) diluted with 1% skim milk-containing TBST, and incubated at room temperature for one hour. The PVDF membrane was washed five times with an appropriate amount of TBST and allowed to develop color with Western Lightning-Plus ECL (Perkin Elmer). The PVDF membrane was photographed by an image analyzer (Bio-Rad) and subjected to densitometric analysis.

Densitometric analysis was carried out in the same manner as in Example 1, and the relative amount of VP1 to the condition in the absence of calcium chloride was calculated.

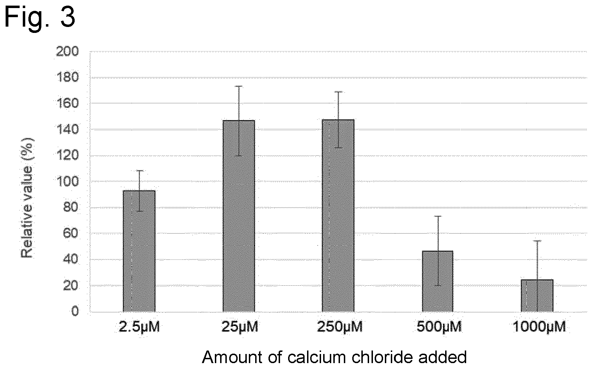

From the analysis result (Figure 3), it was confirmed that the yield of the recombinant protein improved by about 40% at 25 and 250 µM. Here, the yield seems to decrease at 500 and 1 000 µM. However, it is considered that the calcium-dependent protease activity is high due to the increase of calcium ions in the culture medium, and the produced protein of interest is thereby decomposed, and it is considered that the decrease of the expression level of the protein of interest does not result. That is, it is considered that the final concentration of from 25 to 250 µM at which the expression of a protein of interest remarkably increases and proteolysis reaction does not much proceed, is the condition under which a protein of interest can be most efficiently obtained.

## Claims

1. A method for producing a protein in a baculovirus expression system, comprising a step of adding a calcium salt to a culture medium after a recombinant baculovirus is inoculated into an insect cell.

2. The method according to claim 1, wherein the calcium salt is added so as to increase a concentration of calcium in a culture supernatant by from 25 to 250 µM as a final concentration.

3. The method according to claim 1 or 2, wherein the calcium salt is added between 0 and 48 hours after the baculovirus is inoculated.

4. The method according to any one of claims 1 to 3, wherein the calcium salt is calcium chloride.
